# EUROPÄISCHE PATENTANMELDUNG

(11) **EP 2 491 911 A2**
(43) Veröffentlichungstag der Anmeldung: **29.08.2012**
(21) Anmeldenummer: 12460007.3
(22) Anmeldetag: 21.02.2012
(51) Int. Cl.: A61H 3/00, A61H 1/02, A61F 5/01

(54) **Bewegungssystem eines Gerätes für die Unterstützung der Fortbewegung und Rehabilitation, insbesondere für die Rehabilitation von Gehbehinderten.**

(30) Priorität: 28.02.2011 PL 39404911
(71) Anmelder: Frydel, Jaroslaw, Andrzej, 31-610 Krakow (PL)
(72) Erfinder: Frydel, Jaroslaw, Andrzej, 31-610 Krakow (PL)

(57) **Zusammenfassung**

Ein Bewegungssystem der Vorrichtung zur Unterstützung des Gehens und der Gangrehabilitation Gehbehinderter, zur Unterstützung der Fortbewegung des Dauerstehens auch bei körperlich fitten Menschen, gemäß der Erfindungsbeschreibung, mit einer selektiven Verriegelung des Kniegelenks (3) beim Ausstrecken, und Hebung des vorderen Teils des Fußes (4), umfassend: zwei miteinander gelenkig (3) verbundene vertikale Teile (1) und (2), mit zusätzlichen Schellen zur Befestigung des Oberschenkels und des Unterschenkels; ein Federsystem (7) mit einem Verriegelungsboltzen (6), das die beiden vertikalen Teile (1) und (2) zur Geradheit herausfordert; eine Fußplattform (4), die gelenkig mit dem vertikalen Teil (2) verbunden ist; und zwei miteinander gekoppelte Systeme - ein von der Kniebewegung gesteuertes Stößelsystem (10) zur Stabilisierung der Fußlage (4), und ein von der Fußbewegung gesteuertes Zugstabsystem (14) mit der Verriegelung (15) zur Auslösung der Verriegelung (15) des Kniegelenks (5).

## Beschreibung

Die vorliegende Erfindung betrifft ein Bewegungssystem der Vorrichtung zur Unterstützung des Gehens und der Gangrehabilitation Gehbehinderter, insbesondere derjenigen, die an einer Teillähmung einer (Monoparese) oder beider (Paraparese) Gliedmaßen leiden. Das Bewegungssystem der Vorrichtung ist anwendbar u. a. bei der Verbesserung der Gehfähigkeit und Gangrehabilitation von Menschen mit unterschiedlichem Grad der Lähmung der unteren Extremitäten, sowie bei der Unterstützung der Fortbewegung und des Dauerstehens auch bei körperlich fitten Menschen.

Es existiert bereits eine Hilfsvorrichtung für Gehbehinderte, gemäß Patent Nr. 176 092 (PL), dadurch gekennzeichnet, dass sie aus zwei flachen, relativ symmetrisch zueinander gelegenen Vierecken besteht, wobei die oberen Drehachsen der beiden viereckigen Stützelemente mit den Drehachsen von Hüftgelenken übereinstimmen. Jedoch ist die Zwangsimmobilisation des Kniegelenkes aus Sicht der Gangrehabilitation nicht angebracht, weil sie sich sowohl ungünstig auf die Durchblutungsrate des benachbarten Gewebes auswirkt, als auch zur Versteifung des Kniegelenkes und Verschlechterung seines Zustandes führen kann.

Es ist auch eine Hilfsvorrichtung zum Gehen und zur Entspannung für Patienten mit Gehbehinderungen bekannt, gemäß Patent Nr. 195173 (PL), dadurch gekennzeichnet, dass sie aus vertikalen Elementen eines Rahmens mit einer Weste und Fußplattformen besteht, wobei die vertikalen Elemente aus zwei Paaren von Segmenten gebaut sind, von denen jedes Paar Gelenk sowohl miteinander als auch im unteren Teil mit Fußplattformen verbunden ist. Um mit dem Gerät gehen zu können, muss der Benutzer auf den Fußplattformen stehen, sich an speziellen Griffen festhalten und sich von einer zur anderen Seite neigen sowie durch Inanspruchnahme der oberen Extremitäten die Fußplatten abwechselnd heben, um voranzukommen. Diese Hilfsvorrichtung ermöglicht zwar eine Fortbewegung, nutzt aber keine aus der passiven Beugung in Knie- und oberen Sprunggelenken resultierende Energie, die sich jedoch sowohl akkumulieren lässt, als auch später in einzelnen Gangphasen benutzt werden könnte. Außerdem sind die Abmessungen der Fußplattformen beträchtlich größer als die Größe des Schuhwerks, weswegen eine große Beanspruchung der Muskelkraft der oberen Extremitäten beim Gehen zu beachten ist.

Das Bewegungssystem der Vorrichtung zur Unterstützung des Gehens und der Gangrehabilitation Gehbehinderter, gemäß der vorliegenden Erfindung, enthält eine selektive Verriegelung des Kniegelenks und Fußpositionskontrolle, und in der einfachsten Variante besteht aus: zwei gelenkig miteinander verbundenen vertikalen Elementen, an denen sich zusätzliche Schellen zur Befestigung des Oberschenkels (oberes vertikales Teil) und des Unterschenkels (unteres vertikales Teil) befinden; einem Federsystem mit Verriegelung, das die beiden vertikalen Teile zur Geradheit in bestimmten Gangphasen herausfordert; einem horizontalen Element (Fußplattform), das mit dem unteren vertikalen Teil gelenkig verbunden ist; einem von der Kniegelenkbeugung betätigbaren Stößelsystem und einem von der Beugung des oberen Sprunggelenks betätigbaren Zugstabsystem mit einer Verriegelung, wobei das Stößelsystem und das Zugstabsystem mit der Verriegelung miteinander funktionell verbundenen sind.

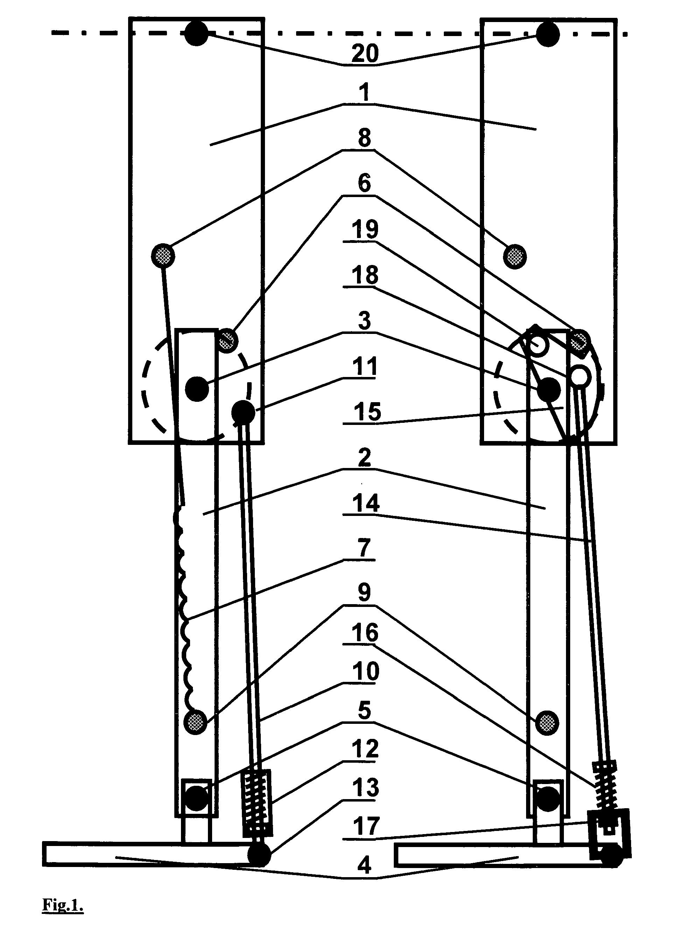

Abbildung Fig.1 veranschaulicht einzelne Teile einer beispielhaften Vorrichtung der vorliegenden Erfindung. Sowohl das obere (1) als auch das untere vertikale Teil (2) werden mit zusätzlichen Schellen zur Befestigung des Oberschenkels (oberes vertikales Teil) und des Unterschenkels (unteres vertikales Teil) ausgerüstet, sodass die untere Extremität umfasst wird sowie eine vollständig synchronisierte Bewegung der miteinander kombinierten Teile möglich ist. Die zusätzlichen Schellen zur Umfassung der Oberschenkel und Unterschenkel werden jeweils an der Seite der vertikalen Teile angebracht. Die Frontaldrehachse (3) des Kniegelenks stimmt mit der Drehachse (3) der Gelenkverbindung zwischen dem oberen vertikalen Teil (1) und dem unteren vertikalen Teil (2) überein. Der Fuß liegt frei auf dem horizontalen Teil - Fußplattform (4). Die Querdrehachse (5) des oberen Sprunggelenks ist zugleich die Drehachse (5) der Gelenkverbindung zwischen der Fußplattform (4) und dem unteren vertikalen Teil (2). Der Umriss der Fußplattform (4) entspricht in etwa der Größe des Benutzerfußes, wobei das vordere Teil der Fußplattform kann auf Höhe des unteren Sprunggelenks flexibel sein.

Das Federsystem mit Verriegelung besteht aus: einer Verriegelung (6), deren Teil im betrachteten Fall ein am oberen vertikalen Teil (1) befestigter Stift ist; einer Feder (7), die in der beispielhafter Vorrichtung eine Kraft zwischen einem am oberen vertikalen Teil (1) angebrachten Stift (8) und einem am unteren vertikalen Teil (2) angebrachten Stift (9) ausübt und die beiden vertikalen Teile zur Geradheit in bestimmten Gangphasen herausfordert; und einer Gelenkverbindung (3). Das drehbare Element (3) der Gelenkverbindung (3) kann sowohl eine Kreisform als auch eine beliebige andere Form besitzen, wodurch das Drehmoment im Kniegelenk variierbar ist. Das drehbare Element (3) kann sich je nach Version synchronisch mit dem oberen (1) oder dem unteren vertikalen Teil (2) bewegen.

Das Stößelsystem und das Zugstabsystem mit der Verriegelung in ihren beispielhaften Versionen werden entsprechend auf der linken und rechten Seite in der Abbildung Fig.1 veranschaulicht.

Das Stößelsystem in einer beispielhaften mechanischen Version besteht aus: einem Stößel (10), der an einem Ende mit dem oberen vertikalen Teil (1), und an dem anderen Ende durch ein federndes System (12) mit der Fußplattform (4) verbunden ist, jeweils mit Hilfe von den Gelenken (11) und (13). Das federnde System ermöglicht eine elastische und reversible Erweiterung des Abstandes zwischen den Gelenken (11) und (13), dank einer zwischen Gehäusedeckel des Stößelsystems und Stößelende wirkenden Federkraft. Das Stößelsystem ermöglicht den vorderen Teil der Fußplattform (4) zu heben, und auch die ganze untere Extremität nach vorne zu neigen.

Das Zugstabsystem mit der Verriegelung in einer beispielhaften mechanischen Version besteht aus: Zugstab (14); Verriegelung (15); einem federnden Einstellsystem (16) mit einem Einstellteil (17); wobei der Zugstab (14) im oberen Teil durch Gelenk (18) mit der Verriegelung (15), und im unteren Teil durch das federnde Einstellsystem (16) mit der Fußplattform (4) gelenkig verbunden ist. Die Verriegelung (15) durch einen Stift (19) am unteren vertikalen Teil (2) drehbar angebracht ist. Eine Sperrkraft, die zwischen dem unteren (2) und dem oberen vertikalen Teil (1) wirkt, entsteht durch Wechselwirkung zwischen der Verrigelung (15) (mit dem unteren vertikalen Teil (2) verbunden) und dem Stift (6) (am oberen vertikalen Teil (1) angebracht). Das federnde Einstellsystem (16) ermöglicht eine elastische und reversible Verminderung des Abstandes zwischen dem Gelenk (18), und einem Gelenk zur Verbindung des federnden Einstellsystems (16) mit der Fußplattform (4). Das Gehäuse des federnden Einstellsystems übt eine von der Gangphase abhängige Kraft aus, die von unten auf den Zugstab (14) wirkt, wobei ein automatisches Einrasten der Verriegelung (15) bei der Wirkung von kleinen Druckkräften mittels des Zugstabs (14) erfolgt. Das Einstellteil (17) bestimmt einen maximalen Winkel zwischen der Fußplattform (4) und dem unteren vertikalen Teil (2), bei dem die Verriegelung (15) ausgelöst wird.

Das Funktionsprinzip der Vorrichtung der vorliegenden Erfindung bezieht sich auf die - wie in den Abbildungen Fig.1, Fig. 2 und Fig.3 dargestellt - beispielhafte Vorrichtung und ist wie folgt: Die gesamte Vorrichtung mit dem Bewegungssystem der vorliegenden Erfindung wird mit Hilfe von zusätzlichen Schellen an einer oder beiden Unterextremitäten befestigt. Nach der Aufrichtung eines Benutzers folgt eine automatische Betätigung des Zugstabsystems (14) und Einrasten der Verriegelung (15) beim gestreckten Kniegelenk (3). Das gesamte Gewicht des Körpers liegt dann auf dem oberen Sprunggelenk. Dank der Betätigung von dem Stößelsystem (10) und dem Zugstabsystem (14) mit der Verriegelung (15) ist Eine Beugung im oberen Sprunggelenk (5) möglich. Eine von der Drehung im oberen Sprunggelenk (5) gesteuerte Auslösung der Verriegelung (15) führt zur Beugung im Kniegelenk (3), wobei diese Beugung durch sowohl das Eigengewicht der unteren Extremität, als auch das Körpergewicht des Benutzers erzwungen wird. Die gesamte Energie der Beugung wird als Federenergie (7) gespeichert. Eine Gleichzeitige Auslösung der Verriegelung (15) und Betätigung des Stößelsystems führt zur leichten Hebung des vorderen Teils der Fußplattform (4). Mit der Beugung der unteren Extremität wird eine Senkung der Hüfte (20) verbunden. Ein kurzweiliges, leichtes Heben der Hüfte führt zu einer selbsttätigen Fortbewegung des Unterschenkels, wobei diese Fortbewegung durch Gravitationsenergie der Unterschenkel, Energie von der Feder (7), Betätigung des Stößelsystems, und die Energie aus der Senkung des vorderen Teil des Fußes zustande kommt. In der letzten Gangphase erzwingt die Feder (7) das größte Streckenmoment im Kniegelenk (3). Eine schnelle Bewegung des Unterschenkels unterstützt das Gehen und die Rehabilitation. Das Einstellteil (16) des Zugstabsystems (14) mit der Verriegelung (15) ermöglicht eine individuelle Anpassung der Schrittlänge. Das Stößelsystem erzwingt eine stabile Schwerpunktlage des Körpers über der von beiden Füßen markierten Fläche beim Gehen und Setzen.

Zu den Vorteilen des Bewegungssystems der Vorrichtung, die Gegenstand der Erfindung ist, gehören unter anderen: Unabhängigkeit von Energiequellen; Bewegungsmöglichkeit aller Gelenke der unteren Extremitäten; Fußbewegungskontrolle im oberen Sprunggelenk; eine von der Fußbewegung gesteuerte selektive Verriegelung des Kniegelenks; Verminderung des für die Bewegung notwendigen Kraftaufwandes durch Speicherung der Energie von der Beugung in Knie- und oberen Sprunggelenken; relativ einfache Bewegungserzwingung und Ausstrecken der unteren Extremität durch Rückkehr (Anheben) der Hüfte in die ursprüngliche Position; eine Bewegung der unteren Extremität(en), die der einer natürlichen nahezu entspricht, während der verschiedenen Gangphasen; Vorbeugung gegen Stolpern durch die Kontrolle der Dorsalflexion des Fußes; eine ermöglichte Sitzposition, ohne das Gerät mit dem Bewegungssystem ablegen zu müssen; stabile Schwerpunktlage des Körpers über der von Füßen markierten Fläche beim Gehen und Sitzen; waagerechte Fußposition beim Sitzen; positive Auswirkung auf die Physiologie eines Menschen mit eingeschränkter Beweglichkeit der unteren Extremität(en).

### Liste der Teile in der Abbildung Fig.1.:

- **1**: Oberes vertikales Teil
- **2**: Unteres vertikales Teil
- **3**: Gelenkverbindung, Frontaldrehachse des Kniegelenks
- **4**: Fußplattform
- **5**: Querdrehachse des oberen Sprunggelenks
- **6**: Verriegelungsbolzen (unbewegliches Teil)
- **7**: Feder
- **8**: Stift am oberen Teil der Feder
- **9**: Stift am unteren Teil der Feder
- **10**: Stößel
- **11**: Das obere Gelenk des Stößels
- **12**: Federndes System des Stößels
- **13**: Das untere Gelenk zur Befestigung des Gehäuses des federnden Systems
- **14**: Zugstab
- **15**: Verriegelung des Kniegelenks (bewegliches Teil)
- **16**: Federndes Einstellsystem des Zugstabs
- **17**: Verschiebbares Einstellteil
- **18**: Gelenk zur Befestigung des Zugstabs am mobilen Teil der Verriegelung
- **19**: Stift - Gelenk zur Befestigung des mobilen Teils der Verriegelung am unteren vertikalen Teil
- **20**: Frontaldrehachse des Hüftgelenks

## Patentansprüche

1. Ein Bewegungssystem der Vorrichtung zur Aufrichtung, Unterstützung des Geradeausgehens und der Gangrehabilitation, **dadurch gekennzeichnet, dass** es umfasst: ein oberes (1) und ein unteres (2) vertikales Teil, wobei die beiden vertikalen Teile miteinander gelenkig (3) verbunden sind; ein horizontales Teil - Fußplattform (4), die mit dem unteren vertikalen Teil (2) gelenkig (5) verbunden ist; und ein Federsystem (7), das die beiden vertikalen Teile (1) und (2) zur Geradheit herausfordert.

2. Ein Bewegungssystem nach Patentanspruch 1, **dadurch gekennzeichnet, dass** es umfasst: ein von der Bewegung des Kniegelenk (3) gesteuertes Stößelsystem (10), gemäß der Beschreibung, das eine Bewegung, bzw. Stabilisierung des Fußes in einzelnen Gangphasen erzwingt.

3. Ein Bewegungssystem nach Patentanspruch 1 oder 2, **dadurch gekennzeichnet, dass** es umfasst: ein von der Bewegung des oberen Sprunggelenks (5) gesteuertes Zugstabsystem (10) mit einem automatischen Einrasten der Verriegelung (15) des Gelenks (3), gemäß der Beschreibung, wobei die Verriegelung (15) automatisch einrastet, wenn die beiden vertikalen Teile (1) und (2) in einer Linie liegen, und die Fußplattformlage neutral ist.

4. Ein Bewegungssystem nach Patentanspruch 3, **dadurch gekennzeichnet, dass** es ein Einstellteil (17) enthält, gemäß der Beschreibung.

5. Ein Bewegungssystem nach Patentanspruch 1 oder 2 oder 3 oder 4, **dadurch gekennzeichnet, dass** das drehbare Element der Gelenkverbindung (3) sich je nach Version synchronisch mit dem oberen (1) oder unteren vertikalen Teil (2) bewegt, und kann sowohl eine Kreisform als auch eine andere Form besitzen, wodurch das Streckenmoment im Kniegelenk variierbar ist.

6. Ein Bewegungssystem nach Patentanspruch 3 oder 3 oder 4 oder 5, **dadurch gekennzeichnet, dass**: das Stößelsystem ein federndes System (12) enthält, wobei das federnde System eine elastische und reversible Erweiterung des Abstandes zwischen den Gelenken (11) und (13) ermöglicht; und das Zugstabsystem mit der Verriegelung ein federndes Einstellsystem (16) enthält, wobei das federnde Einstellsystem eine elastische und reversible Verminderung des Abstandes zwischen dem Gelenk (18), und einem Gelenk zur Verbindung des federnden Einstellsystems (16) mit der Fußplattform (4) ermöglicht.

7. Ein Bewegungssystem nach Patentanspruch 1 oder 2 oder 3 oder 4 oder 5 oder 6, **dadurch gekennzeichnet, dass** sowohl das obere (1) als auch das untere vertikale Teil (2) werden mit zusätzlichen Schellen zur Befestigung des Oberschenkels (oberes vertikales Teil) und des Unterschenkels (unteres vertikales Teil) ausgerüstet, sodass die untere Extremität umfasst wird sowie eine vollständig synchronisierte Bewegung der miteinander kombinierten Teile möglich ist.

8. Anwendung des Bewegungssystems nach Patentanspruch 1 oder 2 oder 3 oder 4 oder 5 oder 6 oder 7 zur Unterstützung des Dauerstehens, auch bei körperlich fitten Menschen,
